# EUROPEAN PATENT APPLICATION

(11) **EP 2 407 543 A1**
(43) Date of publication of application: **18.01.2012**
(21) Application number: 10743436.7
(22) Date of filing: 02.02.2010
(51) Int. Cl.: C12N 15/29, C12N 15/82, A01H 5/00

(54) **USE OF A NUCLEOTIDE SEQUENCE THAT REGULATES FLOWERING TIME, PLANTS EXPRESSING SAME AND PRODUCTION METHOD THEREOF**

(30) Priority: 18.02.2009 ES 200900458
(71) Applicant: Consejo Superior de Investigaciones Cientificas (CSIC), 28006 Madrid (ES); Universidad De Sevilla, 41004 Sevilla (ES)
(72) Inventor: VALVERDE ALBACETE, Federico, E-41092 Sevilla (ES); SERRANO DELGADO, Aurelio, E-41092 Sevilla (ES); ROMERO RODRÍGUEZ, José María, E-41004 Sevilla (ES)
(74) Representative: Fleck, Barbara
(86) International application number: PCT/ES2010/070056
(87) International publication number: WO 2010/094825

(57) **Abstract**

The present invention relates to a nucleotide sequence coding for an amino acid sequence of a unicellular green alga, Chlamydomonas reinhartii, which is expressed in a plant cell from a flowering plant. The expression of the sequence is used to alter the flowering time of a plant that contains the transfected plant cells. In addition, the present invention relates to a method for producing the cells and/or plants of the invention.

SEQUENCE LISTING

<110>  Valverde Albacete, Federico
       Serrano Delgado, Aurelio
       Romero Rodriguez, Jose Maria

<120>  Use of a Nucleotide Sequence that regulates flowering time,
       plants expressing same and production method thereof

<130>  PC926067US

<140>  PCT/ES 2010/070056
<141>  2010-02-02

<150>  P200900458
<151>  2009-02-18

<160>  14

<170>  PatentIn version 3.3

<210>  1
<211>  410
<212>  PRT
<213>  Chlamydomonas reinhardtii

<400>  1

Met Ser Ser Cys Val Val Cys Ala Ala Ala Ala Val Val Trp Cys Gln
1               5                   10                  15

Asn Asp Lys Ala Leu Leu Cys Lys Asp Cys Asp Val Arg Ile His Thr
            20                  25                  30

Ser Asn Ala Val Ala Ala Arg His Thr Arg Phe Val Pro Cys Gln Gly
        35                  40                  45

Cys Asn Lys Ala Gly Ala Ala Leu Tyr Cys Lys Cys Asp Ala Ala His
    50                  55                  60

Met Cys Glu Ala Cys His Ser Ser Asn Pro Leu Ala Ala Thr His Glu
65                  70                  75                  80

Thr Glu Pro Val Ala Pro Leu Pro Ser Val Glu Gln Gly Ala Ala Pro
                85                  90                  95

Glu Pro Gln Val Leu Asn Met Pro Cys Glu Ser Val Ala Gln Ser Ala
            100                 105                 110

Ala Ser Pro Ala Ala Trp Phe Val Asp Asp Glu Lys Met Gly Thr Thr
        115                 120                 125

Ser Phe Phe Asp Ala Pro Ala Val Leu Ser Pro Ser Gly Ser Glu Ala
    130                 135                 140

Val Val Pro Val Met Ser Ala Pro Ile Glu Asp Glu Phe Ala Phe Ala
145                 150                 155                 160

Ala Ala Pro Ala Thr Phe Lys Glu Ile Lys Asp Lys Leu Glu Phe Glu
                165                 170                 175

Ala Leu Asp Leu Asp Asn Asn Trp Leu Asp Met Gly Phe Asp Phe Thr
            180                 185                 190

Asp Ile Leu Ser Asp Gly Pro Ser Asp Val Gly Leu Val Pro Thr Phe
        195                 200                 205

Asp Ala Val Asp Glu Ala Ala Asp Ala Val Ala Asp Ala Ile Val Pro
    210                 215                 220

Thr Phe Glu Glu Glu Gln Pro Gln Leu Gln Gln Gln Glu Pro Leu Val
225                 230                 235                 240

Leu Ala Pro Ala Pro Glu Glu Ser Ala Ala Ser Arg Lys Arg Ala Ala
                245                 250                 255

Ala Glu Glu Ala Ala Glu Glu Pro Ala Ala Lys Val Pro Ala Leu Thr
            260                 265                 270

His Gln Ala Leu Leu Gln Ala Gln Ala Ala Ala Phe Gln Ala Val Pro
        275                 280                 285

Gln Ala Ser Ala Leu Phe Phe Gln Pro Gln Met Leu Ala Ala Leu Pro
    290                 295                 300

His Leu Pro Leu Leu Gln Gln Pro Met Met Pro Ala Ala Val Ala Pro
305                 310                 315                 320

Ala Pro Val Pro Lys Ser Gly Ser Ala Ala Ala Ser Ala Ala Leu Ala
                325                 330                 335

Ala Gly Ala Asn Leu Thr Arg Glu Gln Arg Val Ala Arg Tyr Arg Glu
            340                 345                 350

Lys Arg Lys Asn Arg Ser Phe Ala Lys Thr Ile Arg Tyr Ala Ser Arg
        355                 360                 365

Lys Ala Tyr Ala Glu Ile Arg Pro Arg Ile Lys Gly Arg Phe Ala Lys
    370                 375                 380

Lys Glu Glu Ile Glu Ala Trp Lys Ala Ala His Gly Gly Asp Asp Ala
385                 390                 395                 400

Ile Val Pro Glu Val Leu Asp Ala Glu Cys
                405                 410

<210>  2
<211>  534
<212>  DNA
<213>  Artificial

<220>
<223>  Regulatory sequence of 35S expression

<400>  2
ccctgtcctc tccaaatgaa atgaacttcc ttatatagag gaagggtctt gcgaagctta      60

gtgggattgt gcgtcatccc ttacgtcagt ggagatatca catcaatcca cttgctttga     120

agacgtggtt ggaacgtctt ctttttccac gatgctcctc gtgggtgggg gtccatcttt     180

gggaccactg tcggcagagg catcttcaac gatggccttt cctttatcgc aatgatggca     240

tttgtaggag ccaccttcct tttccactat cttcacaata aagtgacaga tagctgggca     300

atggaatccg aggaggtttc cggatattac cctttgttga aaagtctcaa ttgccctttg     360

gtcttctgag actgtatctt tgatattttt ggagtagaca agcgtgtcgt gctccaccat     420

gttgacgaag attttcttct tgtcattgag tcgtaagaga ctctgtatga actgttcgcc     480

agtctttacg gcgagttctg ttaggtcctc tatttgaatc tttgactcca tggg           534

<210>  3
<211>  2244
<212>  DNA
<213>  Arabidopsis thaliana

<400>  3
aactaggggt gcataatgat ggaacaaagc acaaatcttt taacgcaaac taactacaac      60

cttcttttgg ggtccccatc cccgacccta atgttttgga attaataaaa ctacaatcac     120

ttaccaaaaa ataaaagttc aaggccacta taatttctca tatgaaccta catttataaa     180

taaaatctgg tttcatatta atttcacaca ccaagttact ttctattatt aactgttata     240

atggaccatg aaatcatttg catatgaact gcaatgatac ataatccact ttgttttgtg     300

ggagacattt accagatttc ggtaaattgg tattccccct tttatgtgat tggtcattga     360

tcattgttag tggccagaca tttgaactcc cgtttttttg tctataagaa ttcggaaaca     420

tatagtatcc tttgaaaacg gagaaacaaa taacaatgtg gacaaactag atataatttc     480

aacacaagac tatgggaatg attttaccca ctaattataa tccgatcaca aggtttcaac     540

gaactagttt tccagatatc aaccaaattt actttggaat taaactaact taaaactaat     600

tggttgttcg taaatggtgc tttttttttt tgcggatgtt agtaaagggt tttatgtatt     660

ttatattatt agttatctgt tttcagtgtt atgttgtctc atccataaag tttatatgtt     720

ttttctttgc tctataactt atatatatat atgagtttac agttatattt atacatttca     780

gatacttgat cggcattttt tttggtaaaa aatatatgca tgaaaaactc aagtgtttct     840

tttttaagga atttttaaat ggtgattata tgaatataat catatgtata tccgtatata     900

tatgtagcca gatagttaat tatttggggg atatttgaat tattaatgtt ataatattct     960

ttcttttgac tcgtctggtt aaattaaaga acaaaaaaaa cacatacttt tactgtttta    1020

aaaggttaaa ttaacataat ttattgatta caagtgtcaa gtccatgaca ttgcatgtag    1080

gttcgagact tcagagataa cggaagagat cgataattgt gatcgtaaca tccagatatg    1140

tatgtttaat tttcatttag atgtggatca gagaagataa gtcaaactgt cttcataatt 1200
taagacaacc tcttttaata ttttcccaaa acatgtttta tgtaactact ttgcttatgt 1260
gattgcctga ggatactatt attctctgtc tttattctct tcacaccaca tttaaatagt 1320
ttaagagcat agaaattaat tattttcaaa aaggtgatta tatgcatgca aaatagcaca 1380
ccatttatgt ttatattttc aaattattta atacatttca atatttcata agtgtgattt 1440
tttttttttt tgtcaatttc ataagtgtga tttgtcattt gtattaaaca attgtatcgc 1500
gcagtacaaa taaacagtgg gagaggtgaa aatgcagtta taaaactgtc caataattta 1560
ctaacacatt taaatatcta aaaagagtgt ttcaaaaaaa attcttttga aataagaaaa 1620
gtgatagata tttttacgct ttcgtctgaa aataaaacaa taatagttta ttagaaaaat 1680
gttatcaccg aaaattattc tagtgccact cgctcggatc gaaattcgaa agttatattc 1740
tttctcttta cctaatataa aaatcacaag aaaaatcaat ccgaatatat ctatcaacat 1800
agtatatgcc cttacatatt gtttctgact tttctctatc cgaatttctc gcttcatggt 1860
ttttttttaa catattctca tttaattttc attactatta tataactaaa agatggaaat 1920
aaaataaagt gtctttgaga atcgaacgtc catatcagta agatagtttg tgtgaaggta 1980
aaatctaaaa gatttaagtt ccaaaaacag aaaataatat attacgctaa aaaagaagaa 2040
aataattaaa tacaaaacag aaaaaaataa tatacgacag acacgtgtca cgaagatacc 2100
ctacgctata gacacagctc tgttttctct tttctatgcc tcaaggctct cttaacttca 2160
ctgtctcctc ttcggataat cctatccttc tcttcctata aatacctctc cactcttcct 2220
cttcctccac cactacaacc acca 2244

<210> 4
<211> 18
<212> DNA
<213> Chlamydomonas reinhardtii

<400> 4
agtcgagcag ggcgctgc 18

<210> 5
<211> 19
<212> DNA
<213> Chlamydomonas reinhardtii

<400> 5
gggctcctgc tgctgtaac 19

<210> 6
<211> 20
<212> DNA
<213> Chlamydomonas reinhardtii

<400> 6
attggcggtg gcgacgatgc 20

<210> 7
<211> 20

<212>  DNA
<213>  Chlamydomonas reinhardtii

<400>  7
ggctcgaagg cggcgttggt                                                    20


<210>  8
<211>  20
<212>  DNA
<213>  Arabidopsis thaliana

<400>  8
ggtggagaag acctcaggaa                                                    20


<210>  9
<211>  24
<212>  DNA
<213>  Arabidopsis thaliana

<400>  9
ttatcgcatc acacactata taag                                               24


<210>  10
<211>  30
<212>  DNA
<213>  Arabidopsis thaliana

<400>  10
gatctttgcc ggaaaacaat tggaggatgg                                         30


<210>  11
<211>  30
<212>  DNA
<213>  Arabidopsis thaliana

<400>  11
cgacttgtca ttagaaagaa agagataaca                                         30


<210>  12
<211>  74
<212>  DNA
<213>  artificial

<220>
<223>  Forward primer for amplification of the gene CrCO

<400>  12
ggggacaagt ttgtacaaaa aagcaggctt cgaaggagat agaaccatgt cgagttgcgt        60

cgtgtgcgcg gccg                                                          74


<210>  13
<211>  58
<212>  DNA
<213>  Artificial

<220>
<223>  Reverse primer for amplification of the gene CrCO

<400>  13
ggggaccact ttgtacaaga aagctgggtt cttagcactc agcgtccagg acctcggg          58

<210>  14
<211>  1233
<212>  DNA
<213>  Chlamydomonas reinhardtii

<400>  14
atgtcgagtt gcgtcgtgtg cgcggccgca gcggtcgttt ggtgccagaa tgacaaggcg      60

ctgctttgca aggactgcga tgtgcgcatc cacaccagca acgcggtcgc tgcgcgccat     120

acccgcttcg tgccctgcca gggctgcaac aaggccggtg ctgcgctcta ctgcaagtgc     180

gacgccgcgc acatgtgcga ggcttgccac agctccaacc ccctagctgc tacgcacgag     240

accgagccgg tggcgccgct gccgtcagtc gagcagggcg ctgcaccgga gcctcaggtc     300

ctgaacatgc cctgcgagtc tgtggcgcag tctgcggcca gccccgcggc ttggtttgtg     360

gacgacgaga agatgggcac gaccagcttc tttgatgcgc ctgcggtgct gtcgccctcg     420

ggcagcgagg ccgtggtgcc cgtcatgtcc gcccctatcg aggacgagtt tgcattcgcg     480

gccgccccgg cgacgttcaa ggaaatcaag gacaagctcg agttcgaggc tctggacctg     540

gacaacaact ggctcgacat gggcttcgat ttcactgata tcctgtccga cggcccctct     600

gatgtgggcc tggtccccac cttcgatgcc gtcgatgagg ccgcggatgc cgtggctgac     660

gctatcgtgc ccaccttcga ggaggagcag ccccagttac agcagcagga gcccctggtg     720

ctggctcccg ccccggagga gtcggctgct agccgcaagc gcgctgccgc cgaggaggcc     780

gcggaggagc cggccgccaa ggtgccggcc ctgactcacc aggcgctgct gcaggcgcag     840

gccgccgcct tccaggccgt gccccaggcg tcagcgctgt tcttccagcc gcagatgctg     900

gccgcgctgc cgcacctgcc gctgctgcag cagcccatga tgccggcagc cgtcgccccg     960

gcgcccgtgc ccaagagcgg cagcgccgcc gccagcgcgg ccctcgccgc cggtgccaac    1020

ctgactcgcg agcagcgcgt ggcgcgctac cgcgagaagc ggaagaaccg ctccttcgcc    1080

aagaccatcc gctacgcttc ccgcaaggcg tatgcggaga tccgcccccg cattaagggc    1140

cgcttcgcca agaaggagga gattgaggcc tggaaggcgg cgcacggcgg cgacgacgcc    1200

attgttcccg aggtcctgga cgctgagtgc taa                                 1233

## Description

The present invention relates to a nucleotide sequence coding for an amino acid sequence of a unicellular green alga, *Chlamydomonas reinhardtii,* which is expressed in a plant cell from a flowering plant. The expression of the sequence is used for altering the flowering time of a plant that comprises the transfected plant cells. In addition, the present invention relates to a method for producing the cells and/or the plants of the invention.

### PRIOR ART

The floral transition is a crucial development process for plants because the time at which they reproduce determines the success of the individual and its descendents. To trigger floral transition, plants must coordinate the response to various external and internal signals so as to be able to induce the expression of the genes that induce the transition of the apical meristem from vegetative to reproductive (Bäurle et al., 2006. Cell 125: 655-664). Control of *CONSTANS* (*CO*) at transcriptional and posttranslational level is crucial for photoperiodism, one of the most conserved routes for regulation of floral transition (Kobayashi and Weigel. 2007. Genes Dev. 21: 2371-2384).

The transcription of *CO* in the model plant *Arabidopsis thaliana* is controlled by the circadian clock and the photoperiod, which induces greater abundance of the messenger RNA of CO during the diurnal phase in a long day in summer than in a short day in winter (Suárez-López et al., 2001. Nature 410: 116-1120). These seasonal and daily rhythms of the levels of the *CO* transcript are regulated by a genetic route that includes the genes *GIGANTEA, FLAVIN-BINDING, KETCH REPEAT, F-BOX 1* and *CYCLIN DOF FACTOR 1* (Sawa et al., 2007. Science 318: 261-265). The diurnal accumulation of CO is influenced by the light, under the control of the phytochrome system (Valverde et al., 2004. Science 303: 1003-1006) and is degraded by the proteasome during the night by means of the ubiquitin ligase CONSTITUTIVE PHOTOMORPHOGENIC 1 (COP1), a key regulator of the photomorphogenic development of seedlings (Jang et al., 2008. EMBO J. 27: 1277-1288). These diurnal and nocturnal cycles of degradation of CO, together with the circadian and seasonal regulation of expression of the messenger RNA of CO mean that CO is activated during the evening of a long day. Active CO produces expression of the floral integrator FT in the foliar phloem and the protein FT moves from the leaves to the apical meristem (Corbesier et al., 2007. Science 316: 1030-1033). Once in the apical meristem, FT, together with the transcription factor FD, induces expression of the floral integrators *APETALA1, SUPPRESSOR OF OVEREXPRESSOR OF CONSTANS 1* (*SOC1*)*, LEAFY* and possibly of the floral homeotic genes that control the development of the floral organs.

The CO-FT module is widely distributed in phanerogams and gymnosperms and constitutes one of the most conserved regulatory elements in the induction of flowering (Böhlenius et al., 2006. Science 312: 1040-1043). In all land plants, including the moss *Physcomitrella* (Zobell et al., 2005. Plant Biol. 7: 266-275) and the lycophyte *Selaginella* (this work), there are genes homologous to CO (CO-l*ijkes* or *COLs*)*,* but they do not occur in bacteria, fungi, and animals (Robson et al., 2001. Plant J. 28: 619-631). However, with the exception of some genes of higher plants such as *HEADING DATE 1* (*HD1*) in rice, (*PnCO*) in *Pharbitis nil* or recently that of beet (*BvCOL1*)*,* no *COL* gene of the evolutionary line of plants has been able to complement the co mutation of *Arabidopsis* and therefore display a function in the transition from vegetative to floral phase. The protein COL1, of *Arabidopsis,* with a percentage similarity of 85% with respect to CO, does not have the same function as CO since it cannot complement the co mutant (Ledger et al., 2001. Plant J. 26: 15-22). Previous attempts have failed at the point of demonstrating the presence of true orthology of CO in the moss *Physcomitrella* (Zobell et al., 2005. Plant Biol. 7: 266-275).

*Chlamydomonas reinhardtii* is a unicellular green alga that is used as a model organism owing to its ease of transformation, its robust genetics, its metabolic versatility and its haploid genome, the complete sequence of which was recently released (Merchant et al., 2007. Science 318: 245-251). Certain processes such as phototaxis, synchronous growth and accumulation of starch are regulated by the circadian clock in *Chlamydomonas* (Mittag et al., 2005. Plant Physiol. 137: 399-409). In this and other green microalgae, growth is synchronized with the cell cycle in certain photoperiods, so that the greater part of the population of a culture will be composed, at a given moment, of cells at the same stage of division (Bizova et al., 2005. Plant Physiol. 137: 475-491). A mutant of *Chlamydomonas* (*roc66*) is known that exhibits a somewhat wider period in its growth rhythm than the wild-type alga (Matsuo et al., 2008. Genes Dev. 22: 918-930). Moreover, it has been demonstrated that the circadian clock can permit entry into the cell cycle in *Chlamydomonas* (Goto and Johnson, 1995. J. Cell Biol. 129: 1061-1069) and that the photoperiod also has an enormous influence on this signal and in the progression of the cell cycle in *Ostreococcus tauri,* another green microalga (Moulager et al., 2007. Plant Physiol. 144: 1360-1369).

Bringing forward the production of fruit can represent a commercial advantage. The search for genetic tools making it possible to bring flowering as close as possible to a particular time within the growing cycle, so that collection of the fruit coincides with the most appropriate market period, can mean an increase in yield in the production of trial crops.

### EXPLANATION OF THE INVENTION

The present invention relates to a nucleotide sequence that codes for an amino acid sequence of the unicellular green alga *Chlamydomonas reinhardtii* that is transfected into a plant cell derived from a flowering plant. Gene expression is used for altering the flowering time of a plant that comprises plant cells that express said sequence.

The sequence SEQ ID NO: 1 and the amino acid sequence of the protein CONSTANS (CO) of *Arabidopsis thaliana* have 27% identity and the identity with other COL proteins derived from plants is between 20 and 30%. The low identity does not suggest *a priori* a possible use connected with the function of the aforesaid proteins, especially bearing in mind that proteins such as COL1, whose gene belongs to the same genome of *Arabidopsis thaliana,* has 85% identity with the protein CO and yet does not perform the same function as CO, since it seems not to be able to complement the function of this protein in plants of *Arabidopsis thaliana* that are mutant for *CO* (*co* plants).

Therefore it cannot be deduced, given the low percentage identity mentioned above, that the sequence SEQ ID NO: 1 is functionally comparable to CO, as demonstrated by the fact that it can complement the co mutation and even promote early flowering in *Arabidopsis.* The plant used for carrying out the experiments of complementation and induction of flowering advancement, *Arabzdopsis thaliana,* has been used as a model plant in numerous biotechnological processes, demonstrating that the results obtained can be extrapolated to any other plant species, especially the processes that are conserved in the plant kingdom, as in the case of regulation of flowering by means of the protein CO. The transformation of this plant has been used as an example because the techniques of transformation and monitoring of expression of the recombinant genes have been optimized.

The results obtained in the present invention should not lead to an analysis *ex post-facto,* since from the percentage identity of 27% of SEQ ID NO: 1 with respect to CO we would not expect a result that suggests a possible use of SEQ ID NO: 1 for accelerating the flowering of any plant since it is only by carrying out the transformation of said sequence in the genome of the plant that we can verify the effect on flowering. Finally, the identity of the genes does not make the functionality of SEQ ID NO: 1 obvious.

Application of the use of the sequence SEQ ID NO: 1 of the present invention in various plants whose fruit is of commercial interest may result in substantial advance of flowering and as a consequence may bring forward the production of fruit, offering a commercial advantage over fruit produced by plants that do not comprise the protein of the present invention. Furthermore, stimulating the advance of flowering in the cultivation of plants would make it possible, in principle, to try to approximate flowering as closely as possible to a particular time within the growing cycle, so that subsequent harvesting of the fruit coincides with the most suitable market period, in a given geographic zone. Often this is reflected in increased yield in the production of the crops being tested.

In this connection, the first aspect of the present invention is the use of an isolated nucleic acid comprising a nucleotide sequence that codes for an amino acid sequence with at least 90% identity with the sequence SEQ ID NO: 1, for controlling the flowering time of a plant.

The sequence SEQ ID NO: 1 is an amino acid sequence encoded by a nucleotide sequence present in the genome of the unicellular alga *Chlamydomonas reinhardtii.* The isolated nucleic acid comprises at least one nucleotide sequence that codes for or else a sequence with at least 90% identity with SEQ ID NO: 1 or else that codes for SEQ ID NO: 1. In these aspects of the present invention, the isolated nucleic acid can be the coding nucleotide sequence (cDNA), as in the case of SEQ ID NO: 14, or nucleotide sequences with or without introns, therefore the sequence of genomic DNA that codes for SEQ ID NO: 1 is included (hereinafter, the nucleotide sequences will be referred to as: nucleotide sequences of the present invention or of the invention). That is, they include nucleic acid sequences whose transcription product, messenger RNA (mRNA), codes for the same amino acid sequence (hereinafter, amino acid sequence of the present invention or amino acid sequence of the invention). Variant sequences derived from the nucleotide sequences of the invention, whose product is a protein with the same function as the protein encoded by the sequence SEQ ID NO: 1, are also included. Also amino acid sequences that have modifications at their N-terminal end, C-terminal end and/or in some internal amino acid position so that the function of the protein is the same as results from the translation of the sequence of mRNA transcribed from the nucleotide sequence of the invention, are also included. The amino acid sequence can be encoded by any nucleotide sequence that gives rise to any of the amino acid sequences of the invention. Since the genetic code is degenerated, one and the same amino acid can be encoded by different codons (triplets), therefore the same amino acid sequence can be encoded by different nucleotide sequences.

The amino acid sequences with at least 90% identity with SEQ ID NO: 1 are homologous sequences of other organisms or unicellular algae in which the protein that they encode has an identical function to the protein encoded by said gene. The homologous sequences relate to sequences from different species with similar phenotypic expressions that originate from a common ancestral sequence. Within sequence homology, a distinction is made between two types of homology: orthology and paralogy. Orthologous sequences belong to species that have a common ancestor. Paralogous sequences are those that occur in the same organism and one arises from duplication of the other. In this aspect of the present invention, all homologous sequences are considered, both orthologous and paralogous, that have at least 90% identity with the amino acid sequence SEQ ID NO: 1. Similarly, all those sequences whose transcription product is identical to the amino acid sequence of the present invention are included.

A preferred embodiment is the use of an isolated nucleic acid comprising a nucleotide sequence that codes for an amino acid sequence of a green alga with at least 50% identity with the sequence SEQ ID NO: 1.

The identity of the sequence SEQ ID NO: 1, derived from *Chlamydomonas reinhardtii* with respect to amino acid sequences of other green algae, such as for example, but not limited to, *Volvox,* is of about 60%, therefore this embodiment of the present invention includes any isolated nucleic acid comprising a nucleotide sequence that codes for an amino acid sequence derived from a green alga, with at least 50% identity with respect to the sequence SEQ ID NO: 1.

Another preferred embodiment of the present invention is the use of an isolated nucleic acid comprising a nucleotide sequence that codes for the amino acid sequence SEQ ID NO: 1 for regulating the flowering time of a plant.

As described in the examples section of the present invention, the flowering of model plants of *Arabidopsis thaliana* that have been transformed with a nucleotide sequence that codes for the amino acid sequence of the invention can be brought forward relative to the flowering of control plants that do not contain the sequence SEQ ID NO: 1. Therefore the sequence SEQ ID NO: 1 regulates the flowering time of a plant. The term "regulation of the flowering time" refers to changes of the moment at which the transformation of the vegetative apical meristem to floral meristem occurs, i.e. the moment of flowering. Preferably the nucleic acid sequence of the invention is used for bringing forward the flowering time of a plant.

Yet another aspect of the present invention is the use of an expression vector that comprises the nucleic acid according to any of the aforementioned aspects (hereinafter, vector of the invention) for regulating the flowering time of a plant.

The term "vector" refers to a DNA fragment that has the capacity to replicate in a specified host and, as the term indicates, can serve as a vehicle for multiplying another DNA fragment that has been fused with it (insert). "Insert" refers to a DNA fragment that is fused to the vector; in the case of the present invention, the vector can comprise any of the sequences described according to the preceding aspects that, fused thereto, can replicate in the appropriate host. The vectors can be plasmids, cosmids, bacteriophages or viral vectors, without excluding other types of vectors that correspond to the above definition of vector.

One more aspect is the use of a cell transfected with the vector of the invention that comprises the nucleotide sequence that codes for the amino acid sequence SEQ ID NO: 1 (hereinafter, cell of the invention) for regulating the flowering time of a plant.

The term "cell" in the sense of the present invention refers to a prokaryotic or eukaryotic cell. The cell can be a bacterium capable of replicating a transformed foreign DNA, for example any of the strains of the species *Escherichia coli* or a bacterium capable of transferring the DNA of interest into a plant, for example *Agrobacterium tumefaciens.* Preferably, the cell refers to a eukaryotic plant cell and within this group, more preferably, to those cells belonging to the kingdom *Plantae.* Accordingly, in the case when the cell is a plant cell, the term cell comprises at least one cell of the parenchyma, meristem cell or cell of any type, differentiated or undifferentiated. In addition, this definition also includes a protoplast (plant cell lacking a cell wall).

The term "transfection" refers to the introduction of external genetic material into cells by means of plasmids, viral vectors (in this case it is also called transduction) or other tools for transfer. The term transformation is preferred for describing the nonviral transfers of genetic material into bacteria and nonanimal eukaryotic cells such as fungi, algae or plants.

A preferred embodiment is the use of the cell of the invention that comprises any expression product of the isolated nucleic acid that codes for the amino acid sequence SEQ ID NO: 1.

The term "expression product" in the sense of the present invention refers to any product resulting from expression of the nucleotide sequence according to any of the preceding aspects. Thus, "product resulting from expression of the sequence" means, for example, the RNA that is obtained from transcription of the sequence, the processed RNA, the protein resulting from translation of the RNA or subsequent modifications of the nucleotide sequence within the cell provided that the resultant sequence is derived from the original sequence transferred.

Another preferred embodiment is the use according to any of the preceding aspects or embodiments wherein the isolated nucleic acid is combined functionally with a regulatory sequence of gene expression.

The nucleic acid of the present invention is joined by its 3' end to a regulatory sequence of gene expression. In the present invention, the term "regulatory sequence of gene expression" refers to a nucleic acid sequence that has an effect on the functionality of the nucleic acid sequence of the invention referred to at the beginning of transcription of the DNA sequence or at the start of translation of the RNA sequence or other sequences not described. As an example, the regulatory sequences of gene expression considered in the present invention include promoters and others that are less common, such as certain introns.

According to a preferred embodiment, the regulatory sequence of gene expression is SEQ ID NO: 2 or SEQ ID NO: 3.

The sequence SEQ ID NO: 2 refers to the sequence of the promoter CaMV 35S, which produces constitutive expression of the gene that it precedes in any tissue of the plant. The sequence SEQ ID NO: 3 refers to part of the regulatory sequence of gene expression of the *SUC2* gene, which codes for a sucrose-transporting protein. This sequence causes the preceding gene to be expressed in the accompanying cells or companion cells of the vascular tissue of the phloem.

According to another preferred embodiment, the plant whose regulation of flowering time is referred to according to any of the preceding aspects or embodiments, belongs to the species *Arabidopsis thaliana.* According to another preferred embodiment, the plant belongs to the species *Solanum lycopersicum.*

Another aspect of the present invention is an isolated cell transfected with an isolated nucleic acid comprising a nucleotide sequence that codes for an amino acid sequence with at least 90% identity with the sequence SEQ ID NO: 1.

The term "transfection" refers to the introduction of external genetic material into cells by means of plasmids, viral vectors (in this case it is also called transduction) or other tools for transfer. The term transfection for nonviral methods is used in reference to mammalian eukaryotic cells, whereas the term transformation is preferred for describing nonviral transfers of genetic material into bacteria and nonanimal eukaryotic cells such as fungi, algae or plants. In the case of the present invention, the term transfection is equivalent to the term transformation.

According to a preferred embodiment, the cell comprises a nucleotide sequence of a green alga that codes for an amino acid sequence with at least 50% identity with the sequence SEQ ID NO: 1.

Another preferred embodiment is an isolated cell transfected with an isolated nucleic acid comprising a nucleotide sequence that codes for the amino acid sequence SEQ ID NO: 1.

The aforementioned cells will be referred to hereinafter as the cells of the invention or the cells of the present invention.

The term "isolated cell" refers to a cell that is obtained from any type of microbiological culture (for example, but not limited to, *E. coli* or *Agrobacterium tumefaciens*) or plant tissue. Preferably the cell is a eukaryotic cell derived from a flowering plant. Thus, the term cell comprises at least one cell of the parenchyma, meristem cell or cell of any type, differentiated or undifferentiated. In addition, this definition also includes a protoplast (plant cell lacking a cell wall). The plant cell comprises the sequence SEQ ID NO: 1 stably or transiently.

Yet another aspect of the present invention is a plant that comprises the cell of the invention. According to a preferred embodiment, the plant belongs to the species *Arabidopsis thaliana.* According to another preferred embodiment, the plant belongs to the species *Solanum lycopersicum.* Hereinafter, we shall refer to these plants by the term "the plants of the invention" or "the plants of the present invention".

The term "plant" encompasses every part thereof, that can be stored or cultivated in isolated form or in combination, as well as the germplasm. The germplasm is defined as the biological material that contains the intraspecific genetic variability or as the genetic material that can perpetuate a species or a population of an organism (see below: seeds, propagules or progeny). The plant must comprise the cell of the present invention in such a way that it is expressed in a specific tissue (at a concrete moment of vegetative development or depending on the environmental conditions in which it develops) or constitutively or ectopically (which is expressed in other cells or tissues different from the usual and expected ones).

Taking into account that the protein encoded by the sequence of the present invention performs, surprisingly, a function that restores the function of the CO gene of plants of *Arabidopsis thaliana* whose CO gene has been removed from the genomic sequence (co mutant), it is to be assumed that the transfer and insertion, into the appropriate place, of the sequences of the invention in any plant species where the product obtained from the above sequences has the same amino acid sequence, and as a result gives rise to plants that functionally express these sequences, which perform the same function as the intrinsic CO gene of said plants. In this sense, preferably, the plant is selected from the families of plants of the list that comprises, but is not limited to, the families *Poaceae, Fabaceae, Lauraceae, Chenopodiaceae, Brassicaceae, Cucurbitaceae, Apiaceae, Solanaceae, Asteraceae, Annonaceae, Ebenaceae, Moraceae, Cactaceae, Rosaceae, Rutaceae, Vitaceae, Actinidiaceae, Bromeliaceae, Musaceae, Fagaceae, Betulaceae, Juglandaceae, Anacardiaceae, Oleaceae* or *Capparaceae.*

Preferably, the plant of the family *Cucurbitaceae* is selected from, but is not limited to, the genera *Cucurbita, Cucumis, Citrullus* or *Lagenaria.*

Preferably, the plant of the family *Solanaceae* is selected from, but is not limited to, the genera *Solanum* or *Capsicum.* Preferably the plant of the genus *Solanum* is selected from, but is not limited to, the species *S. tuberosum, S. lycopersicum* or *S. melongena.* More preferably the plant is of the species *S. lycopersicum.* Preferably the plant of the genus *Capsicum* is selected from, but is not limited to, the species *C. angulosum, C. annuum, C. pendulum* or *C. minimum.*

Preferably, the plant of the family *Rosaceae* is selected from, but is not limited to, the subfamilies Rosoideae, *Maloideae* or *Prunoideae.*

Preferably, the plant of the family Rutaceae is selected from, but is not limited to, the genus *Citrus.* More preferably the plant is *C. aurantium, C. nobilis, C. grandis, C. limetta, C. limon, C. medica* or *C. paradisi.*

The plant of the invention can contain any of the sequences of the invention in homozygosity, heterozygosity or hemizygosity.

The plant of the invention can be obtained by genetic transformation of plant cells mediated by biolistics, *Agrobacterium tumefaciens* or any other technique that permits the integration of any of the sequences of the invention in the DNA of the plant, whether genomic, chloroplastic or mitochondrial followed by a program of regeneration *in vitro* suitable for the characteristics and requirements of the plant species transformed. In addition, the plant can also be obtained by transfer of any of the sequences of the invention by crossing, i.e. using pollen of the plant of the invention for pollinating any other plant that does not contain any of the sequences of the invention or pollinating the gynecia of plants that contain any of the sequences of the invention with other pollen of plants that do not contain these sequences. The methods for obtaining the plant of the invention are not limited exclusively to the methods described in this paragraph. In addition, the plant that comprises the cell of the present invention in stable or transient form is also included.

Pollen, seed, propagule, progeny or plant part that is derived from any of the plants described in the present invention.

In the present invention, pollen is taken into account as the transmitter of the genetic and phenotypic characters, which can be carried out by the pollination of any plant variety compatible with the pollen referred to. In this way a plant is obtained that comprises any of the sequences of the present invention and, after respective crossing and/or selection, a plant can be obtained in which the sequence is integrated stably (although they can also be expressed transiently) and in a copy number suitable for obtaining the same desirable characters in the subsequent generations.

Propagules are plant parts that permit propagation or asexual reproduction in plants, by which new plants or individualized organs are obtained. The tissues of the separated portion must recover the condition of meristems for producing the whole set of organs of the plant. The propagule is selected, but not exclusively, from the list comprising stolons, rhizomes, tubers or bulbs.

The term "progeny" refers to the result of reproduction, i.e. the individual or individuals produced by the intervention of one or more parent individuals. For example, the progeny of plants, obtained by sexual reproduction, are the seeds, however the progeny of a plant can be any cell resulting from the fusion of any cellular contents, plastid, cellular compartment, DNA or any combinations thereof. In the processes of cell division (for example when grown *in vitro*) the progeny are the cells resulting from division.

Another aspect of the present invention is a method for obtaining the cell of the invention, which comprises:
a. inserting the nucleotide sequence that codes for SEQ ID NO: 1 in a vector,
b. transforming a cell with the vector obtained according to section (a) and
c. selecting the cell transformed according to section (b) that comprises the nucleotide sequence that codes for SEQ ID NO: 1.

The insertion of any of the sequences of the invention in a vector can be carried out by means of the methods of cloning that form part of common general knowledge, by cutting the sequences and the vector with restriction enzymes and subsequent ligation thereof, so that the sequence of the vector integrates the selected sequence of the invention. The vector was defined in a preceding paragraph.

Selection of the vector that comprises the selected sequence of the invention can be carried out by techniques such as:
- Selection of cells that contain the vectors of the invention by adding antibiotics to the culture medium. The resistance of these cells to substances such as antibiotics is produced by the synthesis of molecules encoded by a sequence contained in the sequence of the vector.

- Digestion with restriction enzymes, by means of which a fragment of some of the sequences of the invention inserted in the vector is obtained.
- Detection of a marker gene present in the transformation vector, whose presence in the plant indicates the presence of the sequences of the invention.

The cell is obtained from any type of microbiological culture (for example *E. coli* or *Agrobacterium tumefaciens*) or plant tissue.

The genetic transformation of the cells is carried out by techniques that form part of common general knowledge, for example electroporation, genetic transformation mediated by biolistics, *Agrobacterium tumefaciens* or any other technique that permits the integration of any of the sequences of the invention into the DNA of the cell. Using these techniques, it is possible to introduce, stably, a vector that includes any of the sequences of the invention, so that, after successive cell divisions, the sequence incorporated is still expressed. Cells that comprise any of the sequences of the invention transiently are also included.

The cell transformed with a vector that includes any of the sequences of the invention can incorporate the sequence in any of the DNAs of the cell: nuclear, mitochondrial and/or chloroplastic (in this case it is usual to insert a sequence of tDNA that contains, among other sequences, any of the sequences of the invention), or remain as part of a vector that possesses its own machinery for self-replicating. Selection of the cell that has incorporated any of the sequences of the invention is carried out by adding antibiotics to the culture medium that supplies nutrients to them. The resistance of these cells to substances such as antibiotics is produced by the synthesis of molecules encoded by a sequence contained in the sequence of the vector or of the tDNA of the vector. The cell that comprises SEQ ID NO: 1 can also be selected by any other technique that permits its presence or absence and/or its expression to be discriminated.

Another aspect of the present invention is a method for obtaining the plant of the invention that comprises:
a. regenerating at least one plant derived from the cell obtained according to section (c) of the above method,
b. selecting one or more plants regenerated according to section (d) that expresses, at least, the nucleotide sequence that codes for SEQ ID NO: 1 and
c. selecting one or more plants obtained according to section (b) that display an advancement of flowering relative to a control plant.

The cells selected, if they are plant cells, can undergo a program of organogenesis or somatic embryogenesis by which, after the dedifferentiation thereof by a suitable combination of plant hormones and other compounds, a complete plant is produced that contains the genetic material of the original cell from which it originates. Moreover, conditions of light and temperature appropriate to each plant species are required. The plant cells are totipotent, i.e. they contain a complete copy of the genetic material of the plant to which they belong regardless of their function or position therein, and therefore has the potential for regenerating a complete new plant. Once the plant derived from the selected plant cell has been regenerated, an analysis is carried out for the presence and/or expression of the nucleotide sequence that codes for SEQ ID NO: 1 or any other sequence of the present invention (promoter sequence, etc.).

The method further comprises selection of a plant that displays an advance or delay of flowering relative to a control. Preferably plants are selected that display an advancement of flowering relative to the control plants. The control plants are plants that do not contain the sequence of the invention that codes for the amino acid sequence SEQ ID NO: 1. Preferably the control plants contain the rest of the transfer DNA sequence of the vector that was used for inserting the nucleotide sequence of the invention into the plant cell. The control can also be a wild-type plant whose plant material is derived from the transforming plants (that comprise the nucleotide sequence that codes for SEQ ID NO: 1), before being transformed, that went through the same cultivation steps *in vitro* as the plants of the invention or that did not go through these cultivation steps.

According to another preferred embodiment, the nucleotide sequence that codes for the sequence SEQ ID NO: 1 is combined functionally with a regulatory sequence of gene expression. The regulatory sequence of gene expression determines, as described above, the plant tissue in which the sequences of the invention are expressed and/or the moment at which they are expressed.

Throughout the description and the claims the word "comprises" and its variants are not intended to exclude other technical characteristics, additives, components or steps. For a person skilled in the art, other objects, advantages and characteristics of the invention will become clear partly from the description and partly from practical application of the invention. The following drawings and examples are provided for purposes of illustration, and are not intended to limit the present invention.

### DESCRIPTION OF THE DRAWINGS

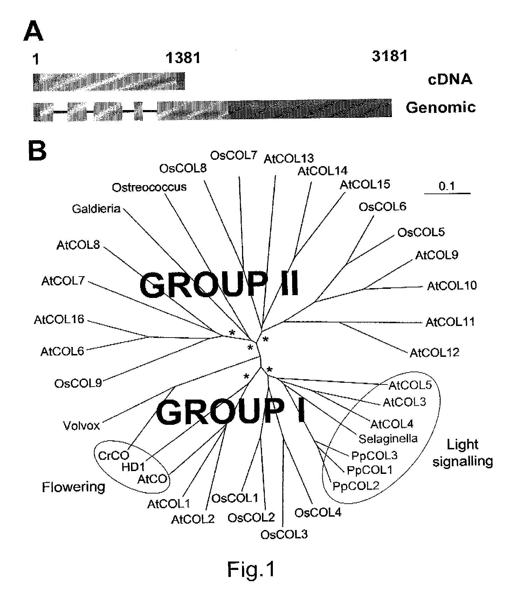

**Fig. 1** **shows the great similarity of SEQ ID NO: 1 relative to the CO proteins of plants**.
   A. The structure of the gene was deduced from the ESTs and amplifications by PCR using template cDNA (top) and the genomic sequence predictable according to *Chlamydomonas genome database* (bottom).
   B. Evolutionary relations of CO and COL proteins of the evolutionary branch of the plants. The distance from the tree is drawn to scale, with the lengths of the branches in the same units as the evolutionary distances used for inferring the phylogenetic tree. The evolutionary relations represent 1000 replicas. The main resultant groups are marked. The asterisks (*) indicate bootstrap values of 95% confidence.

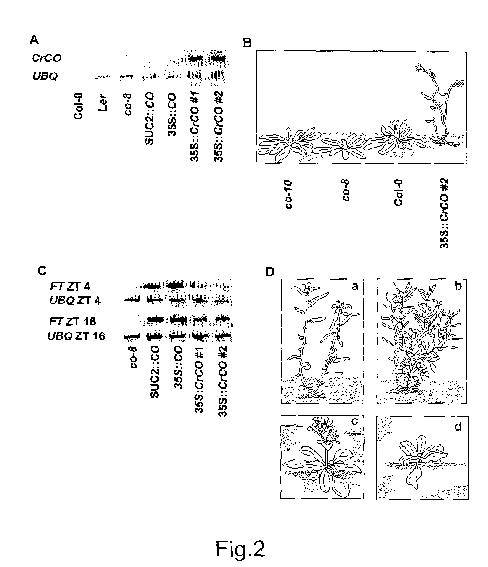

**Fig. 2** **shows the expression of *CrCO* under the control of the 35S promoter.**
   Hereinafter, the amino acid sequence that codes for SEQ ID NO: 1 will be designated *CrCO* and the protein for which it codes will be designated CrCO.
   The expression of *CrCO* under the control of the 35S promoter complements the *co* mutation and accelerates flowering.
   A. Detection of mRNA of *CrCO* by RT-PCR in recombinant plants 35S:: *CrCO,* in plants in pool *co-8* (Ler) (355::CrCO #1) or plants in pool *co*-*10* (col-0) (35S::*CrCO* #2). Wild-type plants col-0 and Ler, as well as the *co-8* mutants were used as negative controls.
   B. Mutant plants *co-8* and *co-10;* col-0 and 35S::*CrCO* #2 were grown in soil for 4 weeks in long-day (LD) conditions. The plants are T3 homozygotes.
   C. Detection of the levels of mRNA of *FT* by RT-PCR in 35S::*CrCO* plants including positive controls (SUC2::*CO* and 35S::*CO*) and negative controls (*co-8*)*.*
      The plants were grown in soil for 2 weeks in long-day conditions and were harvested at midday (ZT 4) and in the afternoon/evening (ZT16).
   D. Phenotype of some 35S::*CrCO* lines.

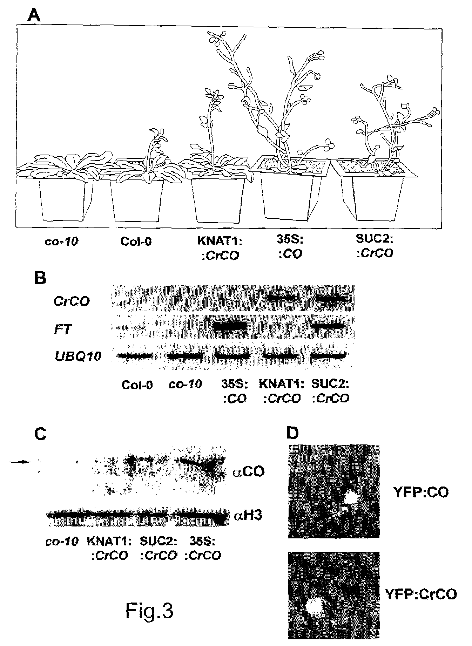

**Fig. 3** **shows the action of CrCO on flowering of Arabidopsis under a phloem-exclusive promoter.**
   A. The expression of *CrCO* under the control of the phloem-specific promoter SUC2 (SEQ ID NO: 3) accelerates flowering in col-0 plants. If expression is controlled by the meristem-specific promoter KNATI, early flowering does not occur. The mutant *co*-*10*, wild-type col-0 and 35S::*CO* plants (in pool col-0) were used as control.
   B. Expression of *CrCO* and FT in the same plants as in A.
   C. Immunodetection of the protein CrCO with antibodies specific to CO in nuclear extracts from plants col-0, 355::*CO* (col-0) and plants col-0 transformed with SUC2:*:CrCO* or KNAT1:*:CrCO*. Detection of H3 was used as load control. The arrow indicates the specific band of 45 KDa detected in plants 35S::*CrCO*, and SUC2::*CrCO.*
   D. Detection of the YFP:CrCO fusion in nuclei of epidermal cells of onion by confocal microscopy.

### EXAMPLES

The invention will be illustrated below by some tests that describe obtaining a nucleotide sequence that codes for the amino acid sequence SEQ ID NO: 1, the method for the production of cells and plants that express it, as well as the use of said sequence for regulating the flowering time of the plants that express it.

### EXAMPLE 1. Experimental protocols

### 1.1 Experimental material from plants and algae

For the analysis of gene expression and production of proteins, the *Arabidopsis thaliana* plants were grown in different photoperiods in phytotrons with controlled conditions of 80% humidity and 75 µE/m² of light intensity. The Arabidopsis plants were also grown in MS-agar solid medium supplemented with 1% (w/v) of sucrose in different photoperiods in an SG-1400 phytotron (Radiber SA, Spain) at 65 µE/m² of light intensity. The wild-type strains 21gr, the cell wall mutant CW15 and the transgenic lines of *C. reinhardtii* were grown in agitated conical vessels or in aerated cylindrical bottles in Sueoka medium or TAP medium in culture rooms controlled at 22°C. For induction of the promoter NIAI, algae grown up to the middle exponential phase in Sueoka-ammonium medium were collected by centrifugation, were resuspended in Sueoka-nitrate medium and were grown in various culture conditions. Different photoperiods were used, from conditions of LD (16:8) to SD (8:16) with lighting conditions of 30 µE/m² (low intensity) or 100 µE/m² (high intensity).

### 1.2 Cloning and analysis of CrCO (amino acid sequence that codes for SEQ ID NO: 1)

Hereinafter, the amino acid sequence that codes for SEQ ID NO: 1 will be designated *CrCO* and the protein for which it codes will be designated CrCO.
Several clones from the cDNA collection of the DNA center of Kazusa were analyzed to obtain the complete ORF of *CrCO.* Sequencing of four of these cDNAs (AV628196; Av628285; AV629179; AV638186) showed a single nucleotide sequence of mRNA. The amino acid sequence deduced from CrCO has 410 residues and three characteristic domains: i) Two zinc-finger domains at the amino-terminal end called b-boxes involved in protein-protein interaction; ii) An intermediate acidic domain possibly involved in transcriptional activation; and iii) A carboxyl domain CCT (CO-COL-TOC1) that has been suggested as mediating in interaction with DNA and that is conserved in plant proteins functionally related with the central oscillator such as *TOC1* or other pseudo-regulators of response.

### 1.3 Analysis of the flowering time

The flowering time was analyzed in plants grown in earth in rooms with LD controlled conditions by counting the rosette leaves and stem leaves of the same stalk in at least 10 individuals. The data are expressed as mean values ± standard error of mean (s.e.m.).

### 1.4 Phylogenetic analyses

The evolutionary relations between the CO and CO-like proteins were analyzed using the amino acid sequences deduced from different databases and aligned with the CLUSTALX program. This alignment was used for generating various evolutionary trees using different phylogenetic criteria. As these trees showed very similar topologies, only that produced with the criterion of minimum evolution is shown (Fig. 1B). The evolutionary relations were calculated using the method of Minimum Evolution (ME). The consensus trapping tree calculated by means of 1000 replicas represents the evolutionary history of the proteins analyzed. The tree is drawn to scale, with the length of the branches in the same units as those of the evolutionary distances used for calculating the phylogenetic tree. The ME tree was traced using the neighbor-joining algorithm at tracing level 1. The neighbor-joining algorithm was used for generating the initial tree. All the positions that contained mismatch gaps or absence of data were removed only in the comparisons of pairing sequences (option of deletion of pairing). In total, there were 669 positions in the set of final results. The phylogenetic analyses were performed with the MEGA4 program. The accession numbers of the sequences used in the alignment are shown in Table 1.

**Table 1. Accession numbers of the COL proteins**

| **Protein** | **NCBI accession number** | **No. of TIGR gene** |
|---|---|---|
| CO | Q39057 | At5g15840 |
| HD1 | NP_910686 | Os06g16370 |
| CrCO | AM940003 | - - |
| AtCOL1 | 050055 | At5g15850 |
| AtCOL2 | Q96502 | At3g02380 |
| AtCOL3 | Q9SK53 | At2g24790 |
| AtCOL4 | Q940T9 | At5g24930 |
| AtCOL5 | Q9FHH8 | At5g57660 |
| AtCOL6 | Q8LG76 | At1g68520 |
| AtCOL7 | Q9C9A9 | At1g73870 |
| AtCOL8 | Q9M9B3 | At1g49130 |
| AtCOL9 | Q9SSE5 | At3g07650 |
| AtCOL10 | Q9LUA9 | At5g48250 |
| AtCOL11 | 023379 | At4g15250 |
| AtCOL12 | Q9LJ44 | At3g21880 |
| AtCOL13 | 082256 | At2g47890 |
| AtCOL14 | 022800 | At2g33500 |
| AtCOL15 | Q9C7E8 | At1g28050 |
| AtCOL16 | Q8RWD0 | At1g25440 |
| OsCOL1 | XP_473042 | Os04g42020 |
| OsCOL2 | XP_506861 | Os02g39710 |
| OsCOL3 | BAD37550 | Os06g44450 |
| OsCOL4 | BAD27992 | Os02g08150 |
| OsCOL5 | NP_001057441 | Os06g19444 |
| OsCOL6 | XP_467550 | Os02g49230 |
| OsCOL7 | BAA33200 | Os03g22770 |
| OsCOL8 | NP_910981 | Os07g47140 |
| OsCOL9 | XP_469510 | Os03g50310 |
| PpCOL1 | AB185925 | - - |
| PpCOL2 | P000371 | - - |
| PpCOL3 | P012466 | - - |
| SmCOL | C_1160095 | - - |
| VcCOL | C_10319 | - - |
| OtCOL | C_Chr_00010305 | - - |
| GsCOL | GS55410 | - - |

### 1.5 Analysis of gene expression

The total RNA derived from cells of Chlamydomonas or seedlings of Arabidopsis was extracted by the TRIZOL protocol (Invitrogen). Gene expression was monitored by *Northern blot* analysis using radioactively labeled probes or by semiquantitative RT-PCR using specific primers for each gene analyzed and cDNA constructed with the *QTAGEN Quick* prime *RT Kit.* The expression levels of the genes of α*-TUBULTN* (*TUA*) or *UBIQUXTIN-10* (*UBQ*) were used as control in Chlamydomonas and Arabidopsis respectively. For circadian gene expression, the expression levels of the gene *CABII,* which codes for the binding protein to chlorophyll a/b of photosystem II of Chlamydomonas, were used as control. The data are shown as the mean of at least three different experiments. The complete list of primers used in this work is shown in Table 2.

**Table 2. List of primers and PCR conditions**

| Gene | Primers | Product (bp) |
|---|---|---|
| *CrCO* | SEQ ID NO: 4 (forward) | 447 |
| | SEQ ID No: 5 (reverse) | |
| *TUA1* | SEQ ID NO: 6 (forward) | 770 |
| | SEQ ID NO: 7 (reverse) | |
| *FT* | SEQ ID NO: 8 (forward) | 560 |
| | SEQ ID NO: 9 (reverse) | |
| *UBQ* | SEQ ID NO: 10 (forward) | 483 |
| | SEQ ID NO: 11 (reverse) | |

For the *CrCO* gene, the PCR conditions are 30 cycles of 30 seconds at 94°C, 30 seconds at 65°C and 60 seconds at 72°C. For the *TUA1* gene, the PCR conditions are 27 cycles of 30 seconds at 94°C, 30 seconds at 65°C and 60 seconds at 72°C. For the FT gene, the PCR conditions are 30 cycles of 30 seconds at 94°C, 30 seconds at 60°C and 60 seconds at 72°C. For the *UBQ* gene, the PCR conditions are 20 cycles of 30 seconds at 94°C, 30 seconds at 65°C and 60 seconds at 72°C.

The name of the proteins as they appear in the corresponding figures is shown in column 1 of Table 2. Column 2 shows the accession number for the proteins in the database of the *National Center for Biotechnology Information* (NCBI), or genomic sequence code of the *DOE Joint Genome Institute* (JGI) (http://www.jgi.doe.gov/) for SmCOL, VcCOL, OtCOL. The amino acid sequence of GsCOL can be downloaded from the website of the genome project of *Galdieria sulphuraria* (http://genomics.msu.edu/giadieria/). Column 3 shows, for Arabidopsis and rice, the accession number of the gene of the *Institute for Gnomic Research* (TIGR).

### 1.6 Immunological and protein analysis techniques

The proteins were extracted from frozen plant material by trituration in a mortar in the presence of liquid nitrogen and an extraction buffer: Tris HCl 50 mM (pH 8.0); EDTA 1 mM (pH 8.0); glycerol 10% (v/v); KCl 50 mM; MgCl₂ 10 mM; PMSF 10 mM and a mixture of protease inhibitors from SIGMA. The algae were disrupted with the same buffer by brief sonication (2 x for 30 seconds in a Branson sonicator at 10 W of power). The resultant raw extract from plants and algae was centrifuged at 16 000 rpm for 30 minutes at 4°C and the supernatant was collected. The precipitate was used for starch measurement. Analysis by *Western blot* used antibodies against CO and antiH3, available commercially (AbCAM), as nuclear controls.

### 1.7 Transformation of algae and plants

The wild-type and mutant plants of Arabidopsis were transformed by the floral dip method mediated by *Agrobacterium.* For the expression of *CrCO* in Arabidopsis, the plants were transformed with the vector *alligator 2.* The CrCOox plants were selected by the presence of GFP in the seed under the microscope and their floral phenotype in the first generation was analyzed. The homozygous plants were selected directly in this generation when they displayed 100% fluorescence in the siliques.

The plasmid *pENSG-YFP:GW* (from Dr. Nieves Medina-Escobar, University of Málaga) was used for generating fluorescent fusion proteins for determining the nuclear localization. This plasmid generates an amino-terminal fusion of the YFP protein to proteins expressed by cDNAs cloned at a *gateway* site at the 3' end of the *YFP* gene, under the control of a 35S promoter and are compatible with Agrobacterium-mediated expression in plants.

For construction of the nitrate-inducible *CrCO-*expressing vector, the C-standard Gateway cassette (Invitrogen) was introduced into the *EcoRV* site of the polylinker of the *pNIA1* vector according to the manufacturer's instructions, obtaining the vector *pNTAG.* A *Hind*III fragment of the p*Hyg3* vector, which includes the hygromycin resistance cassette, was introduced into the HindIII site of the p*BS SK+* vector and a *kpn*I fragment into the same site of the p*NIAG* vector, producing the p*NIAHG* vector, which was confirmed as being cloned in antisense to the Gateway cassette by sequencing. The ORF of *CrCO* was amplified by PCR and was cloned into the p*DONR221* vector by recombination. The primers used were SEQ ID NO: 12 (forward) and SEQ ID NO: 13. By means of this amplification, a cDNA sequence was obtained, which is shown in SEQ ID NO: 14 (*CrCO*)*.* The *CrCO* insertion vector was used as substrate for a reaction L of recombination in the p*NIAHG* vector, obtaining the final vector p*NIAHG::CrCO.* The transformed algae were selected by hygromycin resistance in Sueoka-ammonium medium and expression of the gene was verified in the presence of nitrate.

The vector for production of GST:CrCO was constructed by molecular recombination of the *CrCO* insertion vector and the p*DESRI5* plasmid (Znvitrogen). Cloning and purification in glutathione-sephadex columns (GE Healthcare) was carried out as described by the manufacturer.

### EXAMPLE 2. Results

### 2.1. Identification of a homolog of CONSTANS in C. reinhardtii

The databases of ESTs (Expression Sequence Tags) often contain ESTs of *CrCO,* but only one EST could be identified for the gene *COL* (*CONSTANS-LIKE*) EDP03468. Moreover, using primers designed for an internal part of the gene and, as template, cDNA from different growth conditions, no signal from EDP03468 could be amplified by reverse transcription PCR (RT-PCR). However, expression of *ROC66* has been described as being slight but detectable and appears to be regulated by the circadian clock. In view of its great sequence divergence with CO, and its similarity to proteins of the COL family, *ROC66* could be regarded as a *COL* gene of algae. Significantly, in all growth conditions investigated, the complete sequence of *CrCO* could be amplified by RT-PCR tests, generating a fragment of some 1.4 kb, which is consistent with the size envisaged for the gene in the latest version of the genomic sequence of *C. reinhardtii* (Fig. 1A). The structure of the *CrCO* gene was generated on the basis of information gathered from the ESTs, from the RT-PCR amplifications and from the aforementioned sequence in the database of the genome of *C. reinhardtii* (Fig. 1A). *CrCO* possesses four introns that are conserved in the sequence of the gene of *Volvox* cartieri but not in genes of plants, of the moss *Physcomitrella patens* or of the lycophyte *Selaginella moellendorfi.* The coding sequence of *CrCO* (1230 nt) is similar to that of CO and its mRNA contains short sequences at the 5' and 3' ends. The complete identity of the amino acid sequences deduced from CO and *CrCO* is 27% and the similarity is 37%. In the conserved zone of the b-boxes the identity of amino acids is up to 43%, whereas in the CCT domain the identity reaches 78%. The identities with other COL sequences of plants obtained from databases varied between 20 and 30% (Table 3). Overall, the results strongly suggest that *CrCO* is the only true homolog of *CO* that is normally expressed in *C. reinhardtii.*

**Table 3. Sequence identity of several representative members of the COL family of proteins of the evolutionary tree of the plants**

| | **CO** | **HD1** | **PpCOL1** | **SmCOL1** | **CrCO** | **VcCO** | **OtCOL** | **GsCOL** |
|---|---|---|---|---|---|---|---|---|
| CO | 100 | 46.6 | 34.8 | 36.4 | 26.7 | 26.9 | 24.3 | 23.0 |
| HD1 | | 100 | 33.0 | 32.6 | 26.1 | 25.7 | 22.1 | 21.0 |
| PpCOL1 | | | 100 | 49.4 | 29.1 | 30.9 | 22.5 | 22.7 |
| SmCOL | | | | 100 | 32.5 | 29.4 | 23.4 | 22.2 |
| CrCO | | | | | 100 | 62.2 | 19.8 | 22.2 |
| VcCO | | | | | | 100 | 19.5 | 21.7 |
| Oncol | | | | | | | 100 | 19.0 |
| GsCOL | | | | | | | | 100 |

The numbers indicate the percentage identity of amino acids between each pair of proteins. The identity of CrCO with the sequences of plants is greater than with those of COL proteins of other prasinophyte algae such as *Ostreococcus tauri* (OtCOL) or with the red alga *Galdieria sulphuraria* (GsCOL). PpCOL1: *Physcomitrella patens* COL1; SmCOL: *Selaginella moellendorfi* COL; VcCO: *Volvox cartieri* COL.

The amino acid sequence deduced from CrCO, together with other sequences of COs and COLs of the photosynthetic eukaryotic lineage was used for generating an alignment and an evolutionary tree (Fig. 1B). In the tree in Fig. 1B, CrCO is grouped with Volvox-CO (VcCO), another chlorophyte alga that displays rudimentary multicellular organization. The gene structure and the position of the introns are similar between the two sequences of algae, as was to be expected from the evolutionary proximity of the two species (Fig. 1B). This basal branch is very close to group I, which includes the CO and HD1 proteins, which are known to have a central role in photoperiodic flowering in Arabidopsis and rice, respectively. In contrast, the homologs of CO present in the genome drafts of the unicellular marine green alga *Ostreococcus tauri* and the red alga *Galdieria sulphuraria are* branched far from this group (96% trapping) and together with COL members of group II (Fig. 1B).

COL genes were not found in the genome drafts or databases of ETSs of different species that belong to other taxonomic groups such as the Bacillariophyta (*Thalassiosira pseudonana* and *Phaeodactylum tricornutum*)*,* Dinophyta (*Amphidium operculata*)*,* Euglenophyta (*Euglena gracilis*) or Haptophyta (*Emiliana huxley*)*.* Accordingly, probably the evolutionary branch that finally evolved into the land plants included true homologs of CO, although the sequencing of new genomes of algae might alter this scenario.

### 2.2. Expression of CrCO in Arabidopsis under the control of various promoters accelerates the floral transition

The total cDNA of *CrCO* was cloned under the control of the 35S promoter into an expression vector of plants (see methods). The previous construction, which overexpresses *CrCO,* was transformed into the null mutants *co-8* and *co-10,* as well as into the wild-type ecotypes Ler and Columbia. In the transforming plants, the expression of the *CrCO* gene was measured by RT-PCR (Fig. 2). In all cases expression of *CrCO* under the 35S promoter (35S::*CrCO* plants) complemented the co mutation (Fig. 2A and Fig. 2B) and all the plants showed an early flowering phenotype (Table 4). Conversely, in the transforming plants of Ler and Col-0, a direct co-segregation was observed between the marker of the vector and the early flowering phenotype, demonstrating that CrCO would also confer early flowering in wild-type plants (Table 4).

**Table 4. Flowering time of wild-type, mutant and transgenic plants in long-day (LD) growing conditions**

| **Plant genotype** | **Total number of leaves** |
|---|---|
| | **(± s.e.m.)** |
| Ler | 12.8 ± 0.3 |
| Col-0 | 19.9 ± 0.5 |
| *co-8* (*Ler*) | 33.2 ± 0.7 |
| *co-10* (*col-0*) | 39.7 ± 0.8 |
| 35S::CO (col-0) | 5.8 ± 0.1 |
| SUC2::CO (Ler) | 7.4 ± 0.2 |
| 35S::CrCO #1 (Ler) | 8.4 ± 0.3 |
| 35S::CrCo #2 (co1-0) | 13.2 ± 0.3 |
| SUC2::CrCO (col-0) | 8.6 ± 0.2 |
| KNAT1::CrCO (col-0) | 20.4 ± 0.7 |
| 35S::CrCO #1 (co-8) (Ler) | 10.2 ± 0.2 |
| 35S::CrCO #2 (co-10) (Col-0) | 13.6 ± 0.4 |

The data in the table are shown as the mean ± s.e.m. of 10 plants counted for the total number of leaves in LD conditions. The gene pool of each plant is shown in parentheses alongside each genotype. The transgenic plants described in this work are all T3 plants (of third generation).

It was also verified whether the plants that overexpressed *CrCO* were capable of activating the expression of the main target of *CO,* the floral integrator *FT.* Homozygous 35S::*CrCO* plants were grown in solid medium in LD (16 hours of light and 8 hours of darkness) and the expression of *FT* was analyzed at times ZT 4 and ZT 16 (Fig. 2C). The plants used were wild-type plants, co mutant plants and plants that overexpressed CO (35S::*CO*) as controls. In all cases, the early flowering phenotype of 355::*CrCO*, was paired with high levels of expression of *FT,* even in the morning, when the levels of *FT* are low in the wild-type plants (Fig. 2C). The 35S::*CrCO* plants also flowered early in noninductive SD conditions and this was accompanied by high expression levels of FT. Therefore the early flowering phenotype of the transgenic plants that expressed *CrCO* can probably be attributed to the same process described for the photoperiodic flowering, mediated by *FT.* The 35S::*CO* plants show other pleiotropic phenotypes associated with early flowering such as club-shaped siliques, an increase in male sterility and terminal flowers. Similarly, the 35S::*CrCO* plants had reduced size, defects in the formation of siliques, terminal flowers and often were unable to produce mature seeds, with flowers that degenerated in late development stages (Fig. 2D, a-d). These last-mentioned plants (Fig. 2D, c-d) showed extremely small size, extremely early flowering, assessed both by days of flowering and by total number of leaves, as well as very high levels of mRNA of *CrCO* and *FT.*

CO promotes flowering in LD by expression of a systemic signal in the vascular bundles. To establish whether the expression of *CrCO* specifically in these tissues could also promote flowering, *CrCO* was introduced into col-0 plants of *Arabidopsis* under the control of the sucrose transport promoter *SUC2,* which is known to be expressed preferentially in the companion cells of the vascular tissue of the phloem. The expression of *CrCO* under the promoter *SUC2* (SUC2::*CrCO* plants) induced early flowering in the wild-type plants Col-0 (Fig. 3A and Table 4) and complemented the co mutation. The plants transformed with the construction SUC2::*CrCO* had approximately 8.6 ± 0.2 leaves at the time of appearance of the first flower bud in LD conditions, whereas Col-0 showed 19.9 ± 0.5 leaves in the same conditions (Table 4). However, when the cDNA of *CrCO* was expressed under the control of the promoter of the *KNATI* gene, expression of which is exclusive to the apical meristem, and was introduced into Col-0 plants, flowering was unchanged (Fig. 3A and Table 4). The KNATI::*CrCO* plants flowered with 20.4 ± 0.7 leaves (Table 4). To confirm that the early flowering phenotype observed in the SUC2::*CrCO* plants was due to the activation of photoperiodism, the expression of *FT* was verified by RT-PCR. Effectively, compared with the control plants, *FT* was expressed at high levels in these plants both in LD and in SD and it was concomitant with high expression levels of *CrCO* (Fig. 3B). However, although they displayed high levels of CrCO, higher expression levels of *FT* compared with wild-type plants were not observed when *CrCO* was expressed under the control of the promoter exclusive to the meristem (Fig. 3B).

Using immunoblots, it was shown that the plants that overexpressed *CrCO* in pool Co1-0 also showed high levels of production of the CrCO protein in nuclear extracts (Fig. 3C). The presence of the CrCO protein was also tested by immunoblots in plants that expressed *CrCO* under the promoters *SUC2* and *KNATI* in pool Col-0. Consistent with the mRNA data, the protein could be detected in 35S::*CrCO*, and SUC2::*CrCO* plants, but not in the *co-10* mutant or in plants where the expression of *CrCO* was controlled by the promoter *KNATI* (Fig. 3C).

The transformation of plants with vectors that contained translational fusions of CO to the Green Fluorescent Protein (GFP) had shown the nuclear presence of the GFP:CO fusion. *CrCO* was cloned into the plasmid pENSG-YFP:GW that produces an amino-terminal fusion with the yellow variant of GFP (YFP). This vector was introduced by particle bombardment in cells of the epidermis together with a control vector that expressed CO (YFP:*CO*) in the same conditions. As can be seen in Fig. 3D, YFP:*CrCO* was detected by confocal microscopy in the nucleus similarly to the plants that overexpressed the YFP:*CO* fusion, suggesting that CrCO is also localized in the nucleus.

### EXAMPLE 3. CrCO is a true ortholog of CO

Although the protein CrCO maintains about 27% identity with CO, the plants transformed with *CrCO* have a surprisingly early flowering phenotype. In contrast, COL1, a tandem duplication of *CO* in *Arabidopsis,* which conserves more than 85% identity in amino acids with CO cannot complement the *co* mutation if it is expressed under a 35S promoter. This suggests that the structure of CrCO is similar to that of CO and that it is probably involved in the same nuclear complexes that have been proposed based on the transcription of *FT.* The fact that it can complement both the *co-8* mutation in the Ler pool and the *co-10* mutation in the Col-0 pool indicates that CrCO can effectively replace CO in the supramolecular complex that supposedly is involved in the modification of transcription.

In the present invention it has also been shown that, as with *CO,* the expression of *CrCO* under the control of the promoter of the phloem-specific gene, *SUC2,* causes early flowering in *Arabidopsis.* The SUC2::*CrCO* plants showed higher expression levels of *FT* than the wild-type plants even in noninductive SD conditions and this might explain the early flowering phenotype observed. In contrast, the expression of *CrCO* under the specific promoter of the meristem gene *KNATI* does not promote early flowering and expression of *FT* is not detected. This is also the case with the KNATI::*CO* plants.

In conclusion, CrCO is functional when it is expressed specifically in the vascular tissue, which further supports conservation of the function of CO in the homologous gene of plants despite the considerable phylogenetic distance separating them.

The photoperiodic dependence of gene expression and regulation assumes a fundamental process for the photosynthetic organisms since anticipating the diurnal and seasonal light signals permits selection of the best moment of the day or of the year for effecting the phase transitions. In this connection, plants have adapted photoperiodic regulation for several key processes that control development such as dormancy, the formation of branches or the floral transition. In this regulatory pathway, the COL proteins appear to have an important role, since members of this family are regulated by light and the circadian clock. At the same time they are involved in controlling processes regulated by light such as the PHYB-dependent control of elongation of the hypocotyl, branching or the flowering time. Among the many genes that regulate the floral transition, the *CO-FT* module seems to occur in the majority of families of dicotyledons and monocotyledons and is involved in the control of flowering, from herbaceous plants to trees. In contrast to other systems for regulating flowering such as the FLC/vernalization route, which seems specific to some dicotyledons, the COL genes are present in the genomes of the bryophytes such as the moss *Physcomitrella patens,* the lycophytes such as *Selaginella moellendorfi* and several microalgae (described in the present invention).

Complementation of the function of *CO* with *CrCO* in *Arabidopsis* proves surprising because it implies that the *COL* genes have been involved in circadian regulation and the control of reproduction since very early on within the lineage of the chlorophyta and that these characteristics have been preserved during their evolution. The co mutation is not lethal in *Arabidopsis* or in other plants, although it has probably assumed a crucial characteristic in the capacity of plants to adapt to new environments. In *Chlarnydomonas,* the cell cycle and growth are regulated by the circadian clock and the photoperiod, as is demonstrated by the change in the peak of expression of key components of the cell cycle depending on the length of night/day.
Based on the results presented in the present invention, it can be deduced that conservation of the function of CO may have been critical for the ability of plants to respond to external light signals and promote transitions of development.

## Claims

1. The use of an isolated nucleic acid comprising a nucleotide sequence that codes for an amino acid sequence with at least 90% identity with the sequence SEQ ID NO: 1, for controlling the flowering time of a plant.

2. The use of a nucleic acid as claimed in claim 1 that comprises a nucleotide sequence that codes for an amino acid sequence of a green alga with at least 50% identity with the sequence SEQ ID NO: 1.

3. The use of a nucleic acid as claimed in any one of claims 1 or 2, **characterized in that** the nucleotide sequence codes for the amino acid sequence SEQ ID NO: 1.

4. The use of an expression vector that comprises the nucleic acid as claimed in any one of claims 1 to 3.

5. The use of a cell transfected with the expression vector as claimed in claim 4.

6. The use of the cell as claimed in claim 5 that comprises any expression product of the isolated nucleic acid.

7. The use as claimed in any one of claims 1 to 6, **characterized in that** the isolated nucleic acid is combined functionally with a regulatory sequence of gene expression.

8. The use as claimed in claim 7, **characterized in that** the regulatory sequence of gene expression is SEQ ID NO: 2 or SEQ ID NO: 3.

9. The use as claimed in any one of claims 1 to 8, **characterized in that** the plant belongs to the species *Arabidopsis thaliana.*

10. The use as claimed in any one of claims 1 to 8, **characterized in that** the plant belongs to the species *Solanum lycopersicum.*

11. An isolated cell transfected with an isolated nucleic acid comprising a nucleotide sequence that codes for an amino acid sequence with at least 90% identity with the sequence SEQ ID NO: 1.

12. The cell as claimed in claim 11 that comprises a nucleotide sequence of a green alga that codes for an amino acid sequence with at least 50% identity with the sequence SEQ ID NO: 1.

13. The cell as claimed in either of claims 11 or 12 that comprises a nucleotide sequence that codes for the amino acid sequence SEQ ID NO: 1.

14. A plant that comprises the cell as claimed in any one of claims 11 to 13.

15. The plant as claimed in claim 14 that belongs to the species *Arabidopsis thaliana.*

16. The plant as claimed in claim 14 that belongs to the species *Solanum lycopersicum.*

17. Pollen, seed, propagule, progeny or plant part that is derived from any of the plants as claimed in claims 14 to 16.

18. A method of obtaining the cell as claimed in any one of claims 11 to 13, that comprises:
a. inserting the nucleotide sequence that codes for SEQ ID NO: 1 into a vector,
b. transforming a cell with the vector obtained according to section (a) and
c. selecting the cell transformed according to section (b) that comprises the nucleotide sequence that codes for SEQ ID NO: 1.

19. A method of obtaining a plant as claimed in any one of claims 14 to 16 that comprises:
a. regenerating at least one plant derived from the cell obtained according to section (c) of claim 18,
b. selecting one or more plants regenerated according to section (d) that expresses, at least, the nucleotide sequence that codes for SEQ ID NO: 1 and
c. selecting one or more plants obtained according to section (b) that displays advancement of flowering relative to a control plant.

20. The method as claimed in either of claims 18 or 19, **characterized in that** the nucleotide sequence that codes for SEQ ID NO: 1 is combined functionally with a regulatory sequence of gene expression.
